# EUROPEAN PATENT APPLICATION

(11) **EP 2 784 079 A1**
(43) Date of publication of application: **01.10.2014**
(21) Application number: 13004161.9
(22) Date of filing: 23.08.2013
(51) Int. Cl.: C07H 1/08, C07H 3/10

(54) **Method for producing levoglucosan**

(30) Priority: 25.03.2013 LV 130038
(71) Applicant: "Latvian State Institute of Wood Chemistry" Derived public person, 1006 Riga (LV)
(72) Inventor: Zurins, Aivars, LV1029 Riga (LV); Rizikovs, Janis, LV1013 Riga (LV); Zandersons, Janis, LV1069 Riga (LV)
(74) Representative: Dolgicere, Nina

(57) **Abstract**

Invention relates to processing cellulose-containing raw material and namely obtaining levoglucosan from wood. The raw material is treated by 10-40 % sulphuric acid, hydrolysed with superheated steam at a temperature of 110-140°C and a pressure of 0.2- 0.5 MPa. Then the product obtained is washed with demineralised water till pH 3.5. This method yields up to 30.9 % of cellulose.

## Description

### Field of the Invention

The present invention relates to cellulosic material treatment technologies, namely, to methods for producing levoglucosan from wood raw materials.

### Background of the Invention

A method is known for producing of levoglucosan from pyrolytic products, wherein a pyrolysis is carried out, a heating of wood at a high rate of up to 360-400°C in the superheated steam and carbon dioxide flow, the pre-treating of the ground wood with 0.5% sulphuric acid.

The yield of levoglucosan from wood cellulose reaches 30-50% or 12-20% from the lignocellulosic raw material (Pernikis R., Zandersons J., Lazdina B., Proc. 8th Int. Symp. on Wood and Pulping Chemistry, 1995, Helsinki, Finland, v. III, p. 371-376).

To increase the levoglucosan yield (up to 10-13.4% from the oven-dry wood mass), improved pyrolysis processes using a catalyst, for example, copper powder that is mixed with cellulose powder, is developed, and the pyrolysis process is carried out in a non-oxygen atmosphere at a temperature of 280-450°C (Patent CN102020722). The said process allows increasing the yield of levoglucosan, but because the condensate after pyrolysis contains copper atoms, the isolation of levoglucosan itself is a complicated and labour-consuming process.

From the known levoglucosan preparing methods, the closest to the proposed invention is a process, wherein wood in the form of wood chips is treated with mineral acid, namely, 2% sulphuric acid, the obtained cellolignin is washed with water - at first at a temperature of 100°C, then at a temperature of 80°C for 1 h, and then the solution is poured off and the washing process is repeated until the pH 2.9 (SU503906) is achieved.

The known method allows obtaining the condensate for separating impurities-free levoglucosan, although, using the said method, the levoglucosan yield practically is not increased and is about 12-13% from the oven-dry wood mass.

### Detailed Description of the Invention

The object of this invention is to increase the levoglucosan yield.

According to the present invention, the proposed object is achieved by a method for preparing levoglucosan from cellulose-containing raw material, the method comprising the preliminary treating it with mineral acid, hydrolysing, washing, then pyrolysing the obtained product and isolating levoglucosan from pyrolysis products, wherein the pre-treatment of the cellulose-containing raw material is carried out with 10-40% sulphuric acid, but the following hydrolysis is carried out by superheated steam at a temperature of 110-140°C, and the amount of sulphuric acid is 1-7% from the oven-dry cellulose-containing raw material mass.

The object of the invention is achieved by a hydrolysis in superheated steam at a pressure of 0.2-0.5 MPa, and after the hydrolysis stage the washing of the obtained product with demineralised water until pH 3.5 is achieved.

An example of the realization of the method is given below.

Debarked birch wood chips with an average particle size of 10 x 10 x 5 mm and a moisture content of 30% are treated with 30% sulphuric acid solution. The treated material is then loaded in a 10-L reactor and treated with steam at a temperature of 120°C for 60 min. The obtained lignocellulose is washed with demineralised water until pH 3.5 is achieved, and then dried up to the moisture content < 12% and ground until the particle size < I mm is achieved. Lignocellulose is pyrolysed in an entrained flow reactor, using superheated steam as a heat carrier at the lignocellulose:steam ratio 1:3, and the gas-vapour mixture is condensed in a surface condenser. A condensate containing levoglucosan is obtained, and after the evaporation of the solution levoglucosan may be separated by any known method.

| Hydrolysis temperature, °C | Hydrolysis time, min | Yield of lignocellulose, % | Yield of levoglucosan, rel.% | Yield of levoglucosan from wood, rel.% |
|---|---|---|---|---|
| 100 | 60 | 78.0 | 23.4 | 18.3 |
| | 120 | 77.7 | 27.6 | 21.4 |
| | 480 | 68.5 | 25.9 | 17.7 |
| 110 | 30 | 70.7 | 25.2 | 17.8 |
| | 60 | 74.2 | 24.7 | 18.3 |
| 120 | 60 | 61.7 | 36.1 | 22.3 |
| | 120 | 64.9 | 33.32 | 21.6 |
| 130 | 30 | 65.8 | 27.9 | 18.3 |
| | 60 | 63.9 | 31.0 | 19.8 |
| 140 | 60 | 63.8 | 27.6 | 17.6 |
| | 120 | 61.8 | 29.4 | 18.1 |
| 150 | 30 | 59.7 | 26.5 | 15.8 |
| | 60 | 50 | 23.1 | 11.6 |
| 160 | 60 | 54.2 | 6.8 | 3.7 |
| | 120 | 41.4 | 1.9 | 0.8 |

The treatment of cellulose by the proposed method makes it possible not only to isolate the hemicellulose present between the cellulose molecules, but also to decrease the degree of polymerisation of cellulose to 200-300 units. Besides, treating thereby, partial recrystallisation of cellulose, namely, its structural changes occur. In general, the proposed method allows a considerable increase in the levoglucosan yield, namely, up to 30.9% from the cellulose amount.

## Claims

1. A method for producing of levoglucosan from cellulose-containing raw material, the method comprising a preliminary treating it with mineral acid, hydrolysing, then pyrolysing of the obtained product and isolating the levoglucosan from pyrolysis products, wherein the pre-treatment of the cellulose-containing raw material is carried out with 10-40% sulphuric acid, but the following hydrolysis is carried out by superheated steam at a temperature of 110-140°C.

2. A method according to claim 1, wherein after the hydrolysis stage the obtained product is washed with demineralised water until pH 3.5 is achieved.

3. A method according to claim 1, wherein the pyrolysis by superheated steam is carried out at a pressure of 0.2-0.5 MPa.

4. A method according to claim 1, wherein the amount of sulphuric acid is 1-7% from the oven-dry cellulose-containing raw material mass.
